# FASCICULE DE BREVET EUROPEEN

(11) **EP 2 567 733 B1**
(45) Date de publication et mention de la délivrance du brevet: **01.01.2014**
(21) Numéro de dépôt: 12175898.1
(22) Date de dépôt: 11.07.2012
(51) Int. Cl.: A61N 1/375, H01R 13/52, H01R 24/58, B29C 45/14, H01R 43/24, H01R 24/76

(54) **Procédé de réalisation d'une tête de connecteur pour sonde multipolaire de dispositif médical implantable actif**
Herstellungsverfahren eines Verbindungskopfes für eine multipolare Sonde einer aktiven implantierbaren medizinischen Vorrichtung
Method for producing a connector head for a multipolar probe of an active implantable medical device

(30) Priorité: 08.09.2011 FR 1157994
(43) Date de publication de la demande: 13.03.2013
(73) Titulaire: Sorin CRM SAS, 92140 Clamart (FR)
(72) Inventeur: Jullien, Elodie, 69570 Dardilly (FR); Lucas, François, 92120 Montrouge (FR)
(74) Mandataire: Dupuis-Latour, Dominique

(56) Documents cités:
- EP-A2- 2 228 096
- US-A1- 2007 225 772
- US-A1- 2008 234 778
- US-A1- 2009 017 700
- US-A1- 2009 118 778
- US-B1- 7 630 768

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle, ...

Il existe des systèmes de connexion normalisés permettant de garantir l'interchangeabilité des sondes et des générateurs produits par différents fabricants.

Ainsi, les standard dits "IS-1" et "IS-4" définissent un certain nombre de caractéristiques dimensionnelles et électriques relatives aux sondes de délivrance d'impulsions de stimulation (ou de resynchronisation). Pour les sondes de défibrillation, où les contraintes électriques sont plus sévères compte tenu de l'énergie élevée devant transiter du générateur à la sonde, les standards dits "DF-1" et "DF-4" définissent les caractéristiques dimensionnelles et électriques du système de connexion.

La complexité de ces sondes, qui intègrent déjà les contraintes spécifiques en termes d'énergie électrique liées à l'une ou l'autre des utilisations, stimulation ou choc, est encore accrue par le développement des dispositifs multisite et des capteurs intracardiaques, tels que les capteurs d'accélération endocavitaire (EA). Ceci conduit, du point de vue de la connectique, à une multiplication des fiches de connexion, avec au surplus des standards différents selon les fiches.

On comprend l'avantage que peut représenter une fiche unique, soumise à un standard unique, portant une pluralité de contacts électriques permettant d'assurer simultanément l'établissement des connexions aux diverses bornes du générateur pour tous les niveaux d'énergie, qu'il s'agisse du recueil de signaux de dépolarisation, de l'application d'impulsions de stimulation ou de l'envoi d'une énergie de choc de défibrillation.

Dans ce cadre, il a été proposé une fiche de connexion unique du type "isodiamètre", c'est-à-dire de forme cylindrique uniforme, destinée à être insérée dans une cavité cylindrique homologue de la tête de connecteur du générateur.

Le EP 1 641 084 A1 (ELA Medical) décrit une telle fiche de connexion isodiamètre dont la surface cylindrique extérieure présente un empilement de zones de contact électrique annulaires, réalisées par des bagues cylindriques conductrices, et de zones cylindriques isolantes destinées à isoler électriquement les bagues conductrices.

Pour recevoir une fiche de connexion multipolaire de ce type et la connecter aux circuits électriques du générateur, les têtes de connecteur adaptées comprennent une cavité constituée de manière similaire à la fiche elle-même, au sens où elle comporte également un empilement d'éléments annulaires de contact électrique aptes à réaliser un contact électrique avec les bagues conductrices correspondantes de la fiche de connexion. Dans cet empilement, les éléments annulaires de contact électrique sont séparés alternativement par des éléments annulaires d'isolement munis sur leur diamètre intérieur d'anneaux d'étanchéité présentant un profil donné, destinés à appliquer une compression radiale sur les zones cylindriques isolantes de la fiche de connexion.

On remarquera que, contrairement aux réalisations précédentes où les éléments d'isolement étaient portés par la sonde, ces derniers sont maintenant disposés dans la cavité, ce qui présente l'avantage de fournir des éléments d'isolement neufs à chaque fois que le générateur est remplacé. Le terme d"'isolement" employé ici concerne aussi bien l'étanchéité de la cavité par rapport au milieu extérieur, vis-à-vis en particulier des liquides corporels, que l'isolation électrique, c'est-à-dire l'isolation basse tension des zones et des éléments de contact électrique, ainsi que l'isolation haute tension de la cavité avec le milieu extérieur.

Le US 2008/0234778 A1 décrit un procédé de réalisation d'une tête de connecteur, qui prévoit de réaliser une pièce monobloc élastique, en silicone, s'étendant sur toute la longueur de la cavité. Cette pièce en silicone est formée par moulage autour d'une broche centrale de forme appropriée. Pour pouvoir retirer la broche après réticulation du silicone, la pièce est trempée dans un bain de n-heptane, ce qui a pour effet de faire gonfler le silicone et de dilater radialement la pièce, autorisant alors le retrait de la broche de moulage. Cet état dilaté est également utilisé pour insérer par la cavité centrale ainsi dégagée les éléments de contact électrique, dans des logements prévus dans la pièce silicone. Après évaporation du n-heptane, la pièce en silicone reprend ses dimensions originelles, définitives, avec les éléments de contact électrique enchâssés dedans. Mais en raison de la souplesse du matériau élastomère, susceptible de compressions et de détentes, ce procédé connu ne garantit pas la coaxialité des éléments de contact électrique avec les éléments d'isolement, pas plus que leur positionnement axial.

De même, les EP 2 228 096 A2 et US2009/0017700 A1 proposent un procédé de réalisation d'une tête de connecteur en élastomère ou en époxy, dont la cavité intègre des joints en élastomère. Les contacts électriques sont mis en place entre les joints de la cavité à travers des fenêtres latérales ménagées dans la tête de connecteur, ce qui ne permet pas non plus de contrôler la coaxialité des contacts électriques avec les joints en élastomère ni leur positionnement axial. De plus, le problème de l'étanchéité et de l'isolation électrique au niveau de l'interface des joints en élastomère et de la tête n'est pas résolu.

Les US 7 630 768 B1 et US 2009/0118778 A1 décrivent des procédés de réalisation d'une tête de connecteur surmoulée dans une résine dure (typiquement un polyuréthane), permettant de positionner et d'assembler de façon très précise et fiable les éléments de contact électrique. En revanche, ils n'abordent en aucune façon la réalisation d'éléments d'étanchéité souples, pas plus que le problème de l'isolement des éléments de contact électrique entre eux et avec leur environnement, ni celui de l'étanchéité de la cavité en particulier vis-à-vis des liquides corporels. Aussi, un but de l'invention est de proposer un procédé de réalisation d'une tête de connecteur qui permette, de manière simple et économique, d'assurer la coaxialité des éléments de contact électrique et d'isolement ainsi que le positionnement axial des éléments d'isolement.

Ce but est atteint, conformément à l'invention, grâce à un procédé de réalisation par bi-injection d'une tête de connecteur pour un générateur de dispositif médical implantable actif, la tête de connecteur comprenant une cavité sensiblement cylindrique destinée d'une part à recevoir une sonde multipolaire du dispositif et comportant d'autre part une alternance d'éléments annulaires de contact électrique et d'éléments annulaires d'isolement présentant un profil intérieur d'étanchéité, remarquable en ce que ce procédé de bi-injection comprend des étapes consistant à :
- fournir une broche cylindrique de diamètre sensiblement égal au diamètre de la cavité, la broche comportant alternativement des sections lisses destinées à recevoir les éléments annulaires de contact électrique, et des sections profilées portant un profil conjugué du profil d'étanchéité des éléments annulaires d'isolement à réaliser ;
- disposer les éléments de contact électrique sur les sections lisses de la broche en laissant subsister axialement entre ces éléments de contact électrique un intervalle correspondant auxdites sections profilées destinées à la réalisation des éléments annulaires d'isolement ;
- injecter dans un premier moule un premier matériau élastique autour des sections profilées de manière à réaliser les éléments d'isolement ;
- injecter dans un second moule un second matériau, de dureté supérieure à celle du premier matériau, autour de la broche de manière à ancrer définitivement les éléments de contact électrique et réaliser la tête de connecteur ; et
- dégager axialement la broche d'avec la cavité de la tête de connecteur ainsi obtenue.

Ainsi, on comprend que, du fait de leur mise en place préalable directement sur les sections lisses de la broche, les éléments annulaires de contact électrique (ou, plus simplement, les "contacts électriques") sont parfaitement concentriques aux éléments annulaires d'isolement (ou "joints") après que ceux-ci aient été réalisés par bi-injection autour des sections profilées de la broche au moyen du premier matériau élastique, qu'on appellera aussi "matériau souple".

La coaxialité ainsi obtenue à la suite de la première injection de matériau souple est maintenue grâce à l'injection du deuxième matériau de dureté plus élevée, ou "matériau rigide", dans lequel les contacts électriques restent ancrés même lorsque la broche est dégagée axialement par déformation élastique du matériau souple constituant les profils d'étanchéité des joints. Ces profils d'étanchéité sont conçus de sorte à appliquer une compression radiale sur les zones isolantes de la fiche de connexion et assurer ainsi l'étanchéité du connecteur vis-à-vis des fluides corporels tout en contribuant à l'isolation électrique entre deux pôles de la fiche. Avantageusement, le premier matériau déformable présente une dureté inférieure à 80 Shore A. Il peut s'agir d'un matériau élastomère, notamment un composé silicone, ou d'un polymère thermoplastique, notamment un polyuréthane thermoplastique.

De préférence, le second matériau a une dureté supérieure ou égale à 70 Shore D. Ce peut être un polymère thermoplastique, notamment un polyuréthane thermoplastique.

Bien entendu, les matériaux utilisés pour réaliser la bi-injection sont des matériaux biocompatibles.

On remarquera également que le procédé conforme à l'invention assure un positionnement axial des joints parfait, lequel est déterminé de manière définitive par la position des sections profilées le long de la broche. Après la deuxième injection, ce positionnement axial est conservé par adhésion des joints en matériau souple dans le matériau rigide de la tête de connecteur.

Le procédé objet de l'invention offre encore bien d'autres avantages.

En particulier, sa mise en oeuvre industrielle est facilitée par la réduction du nombre de pièces à assembler. En effet, il n'est pas nécessaire de réaliser des joints séparément puis de les monter dans la tête de connecteur, puisqu'avec l'invention ils sont directement intégrés dans la cavité par la bi-injection de matériaux. Le procédé permet donc d'apporter à la fois l'élasticité des joints et la rigidité de la forme extérieure de la tête de connecteur.

Dans le même ordre d'idées, il faut souligner que les profils d'étanchéité portés par les joints sont directement formés sur la broche du moule lors de la bi-injection.

D'autre part, on peut observer que le volume global de la cavité peut être diminué, les divers éléments de la tête de connecteur étant directement intégrés dans la cavité ou moulés ensemble.

Enfin, lors de la bi-injection, les matériaux biocompatibles adhèrent entre eux de façon à obtenir une zone d'adhésion permettant d'assurer l'étanchéité avec le milieu extérieur, et l'isolation des contacts électriques entre eux et avec le milieu extérieur.

Avantageusement, de manière que les contacts électriques puissent occuper la position requise à l'intérieur de la cavité, il est prévu que le procédé de l'invention comporte une étape consistant, avant injection du premier matériau, à contraindre, à l'aide de moyens de positionnement, les éléments de contact électrique dans une position axiale déterminée sur les sections lisses de la broche, cette position étant maintenue durant les étapes d'injection.

On obtient de la sorte un positionnement axial parfait des contacts électriques qui résulte, d'une part, du fait que lors de la première injection les contacts électriques sont parfaitement disposés le long de la broche grâce aux moyens de positionnement, et, d'autre part, du fait que les contacts électriques sont définitivement ancrés lors de la deuxième injection de matériau rigide.

Selon un mode de réalisation particulier, les moyens de positionnement comprennent des rainures annulaires formées sur le diamètre extérieur des éléments de contact électrique et des organes de calage en position des éléments de contact électrique, aptes à coopérer avec les rainures annulaires.

Avantageusement, l'invention propose que le premier moule d'injection soit dimensionné de manière que le diamètre extérieur des éléments d'isolement soit inférieur au diamètre extérieur des éléments de contact électrique.

Cette disposition a pour conséquence qu'après la deuxième injection, les contacts électriques se trouvent encadrés au niveau de leur diamètre extérieur entre deux parois de matériau rigide, ce qui leur garantit un excellent blocage dans le sens axial et donc le maintien de leur positionnement axial initial.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue en perspective d'une tête de connecteur réalisée au moyen du procédé selon l'invention.
La Figure 2a est une vue en perspective d'une broche utilisée lors des étapes de moulage du procédé selon l'invention.
La Figure 2b est une vue de côté de la broche de la Figure 2a.
La Figure 3 est une vue en coupe illustrant les étapes de moulage du procédé selon l'invention.
La Figure 4a est une vue en perspective illustrant la seconde étape de moulage du procédé selon l'invention.
La Figure 4b est une vue en perspective d'une variante de la Figure 4a.
La Figure 5 est une vue en coupe d'une tête de connecteur résultant de la seconde étape de moulage selon les Figures 4a et 4b.

On va maintenant décrire un exemple de mise en oeuvre du procédé de l'invention, en regard des dessins annexés donnés à titre d'exemples non limitatifs.

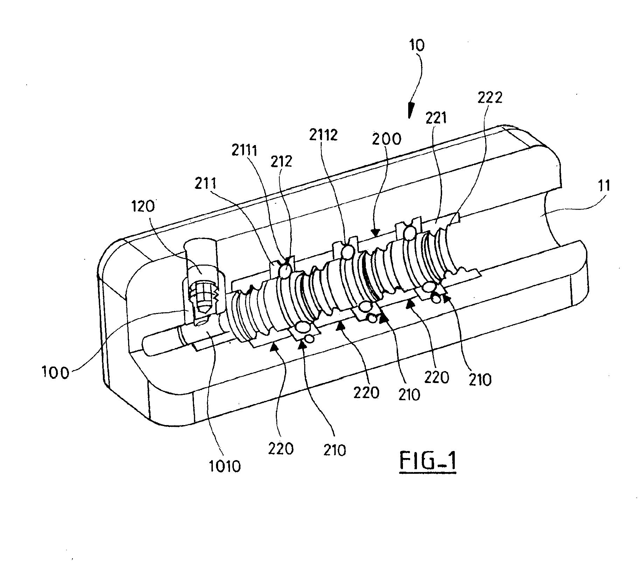

Sur la Figure 1 est représentée une tête de connecteur 10 pour un générateur de dispositif médical implantable actif.

La tête de connecteur 10 comprend une cavité 11 sensiblement cylindrique destinée à recevoir, en conformité avec la norme IS-4/DF-4 (ISO 27186-2010), une fiche de connexion d'une sonde multipolaire (non représentée), de manière à pouvoir échanger des signaux électriques entre le générateur du dispositif et le coeur d'un patient.

Le EP 1 641 084 A1 (ELA Médical) décrit un exemple d'une telle fiche de connexion, à quatre pôles. Plus précisément, cette fiche de connexion connue est du type isodiamètre, présentant à une de ses extrémités une broche de contact électrique axiale et, sur le corps même de la fiche, trois zones de contact électrique annulaires réalisées par des bagues cylindriques conductrices. Les zones de contact électrique annulaires sont séparées alternativement par des zones cylindriques isolantes intercalaires afin d'isoler électriquement les zones de contact électrique les unes des autres.

On peut ainsi, en enfichant d'un seul geste la fiche de connexion dans la cavité 11 de la tête 10, réaliser simultanément toutes les liaisons électriques nécessaires entre le générateur et les pôles de la sonde.

La cavité 11 doit bien entendu être munie à cet effet d'éléments homologues à ceux présents sur la fiche de connexion. Ces éléments vont maintenant être décrits en détail en relation avec la norme précitée. Comme le montre la Figure 1, la cavité 11 de la tête 10 comprend un insert à vis 100 permettant, après que la broche axiale de la fiche de connexion ait été introduite dans une douille 1010 de l'insert, de réaliser la liaison électrique de la broche axiale avec le générateur et de maintenir la fiche dans la cavité 11, au moyen de la vis 120.

La cavité 11 contient en outre une alternance 200 d'éléments de contact électrique et d'éléments d'isolement.

Plus précisément, l'alternance 200 comprend trois éléments de contact électrique annulaires 210 prévus pour réaliser un contact électrique avec les bagues conductrices correspondantes de la fiche de connexion dans le but de transmettre, avec la broche axiale, l'ensemble des signaux électriques haute et/ou basse tension de l'électronique du générateur vers le coeur via la sonde multipolaire implantée et inversement. Ces éléments de contact électrique (ou "contacts électriques") 210 doivent pouvoir recevoir les fiches de connexion des sondes et répondre aux exigences d'effort d'insertion et d'extraction et de performances électriques définies dans la norme.

On peut voir sur la Figure 1 que les contacts électriques 210 sont composés de deux sous-éléments cylindriques annulaires, à savoir une cage 211 en matériau biocompatible tel que l'acier inox MP35N, et un contact élastique 212 reçu par la cage 211 à l'intérieur d'une gorge 2111 ménagée dans sa partie interne, le long du diamètre intérieur.

Le contact élastique 212, tel qu'un ressort cylindrique, se compose d'une seule pièce en métal élastique implantable et réalise une fonction de contact souple avec les bagues conductrices de la fiche de connexion de la sonde, de façon à obtenir le meilleur contact électrique et de faibles résistances de contact.

Le diamètre extérieur de la cage 211 est une zone de connexion électrique des fils reliant les contacts élastiques 212 à l'électronique du générateur, par soudure laser ou électrique.

Comme illustré Figure 1, les contacts électriques 210 sont séparés alternativement le long de l'alternance 200 par des éléments annulaires d'isolement (ou "joints") 220 destinés à être disposés en regard des zones isolantes de la fiche de connexion, de manière à créer une isolation électrique entre les contacts électriques 210 entre eux, et réaliser l'isolement électrique et l'étanchéité de la cavité 11 vis-à-vis du milieu extérieur. Les éléments d'isolement 220 doivent de plus garantir une isolation haute tension et supprimer les chemins de fuite au niveau de leur diamètre intérieur, à l'interface avec les zones isolantes de la fiche de connexion ainsi qu'au niveau de leur diamètre extérieur, à l'interface avec la cavité 11. Comme le montre la Figure 1, les joints 220 se composent d'un corps de joint 221 en matériau biocompatible élastique, comme un élastomère du type silicone, de dureté inférieure à 80 Shore A. La face annulaire interne du corps de joint 221 porte un profil d'étanchéité 222, constitué ici de deux anneaux capables de se comprimer radialement sur une zone isolante de la fiche de connexion lorsque cette dernière est insérée dans la tête de connecteur 10, assurant ainsi l'étanchéité de la cavité 11 et l'isolation entre deux bagues conductrices consécutives de la fiche et les contacts élastiques 212 correspondants.

Tous les éléments de contact électrique et d'isolement décrits ci-dessus sont enrobés ensemble dans la tête 10 de connecteur proprement dite, constituée d'un matériau de dureté supérieure à celle du matériau élastique des joints 220, tel qu'un polyuréthane thermoplastique de dureté supérieure ou égale à 70 Shore D.

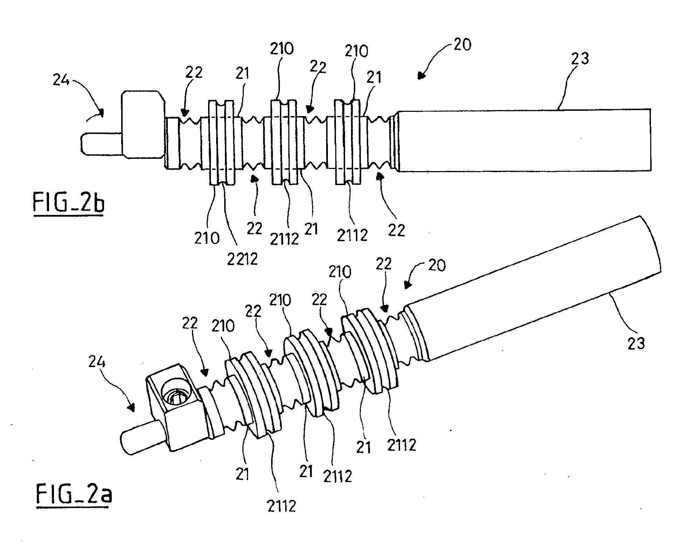

Pour réaliser la tête 10 de connecteur de la Figure 1, l'invention propose un procédé de moulage mettant en oeuvre une broche 20 représentée sur les Figures 2a et 2b.

Comme on peut le voir sur ces figures, la broche 20, de forme générale cylindrique avec un diamètre sensiblement égal à celui de la cavité 11, est segmentée en une pluralité de sections associées aux différents éléments fonctionnels de la cavité 11.

L'extrémité proximale 23 de la broche 20 correspond à l'entrée de la cavité 11, tandis que son extrémité distale 24 est équipée des composants nécessaires au moulage de l'insert à vis 100.

La portion de la broche 20 située entre ses extrémités proximale 23 et distale 24 est divisée en une alternance de sections lisses 21 et de sections profilées 22. Sur les sections lisses 21 sont disposés les contacts électriques 210 décrits plus haut, tandis que les sections profilés 22 portent un profil conjugué du profil 222 d'étanchéité des joints 220.

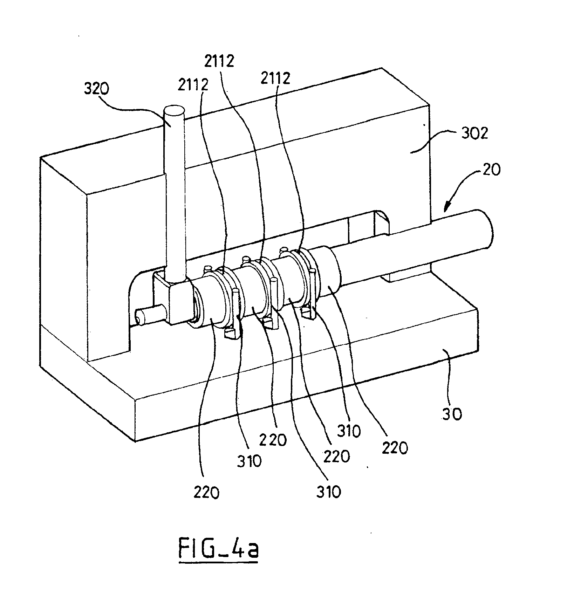

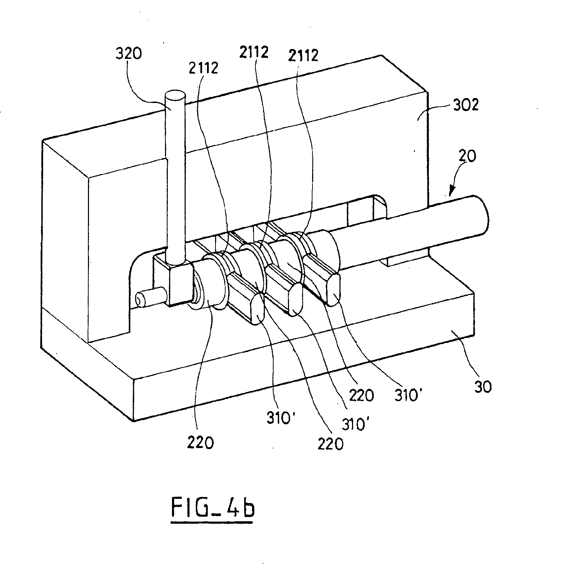

La broche 20 ainsi constituée est placée sur une platine 30 de presse à injection comprenant des organes de calage en position des contacts électriques 210, aptes à coopérer avec des rainures annulaires 2112 formées sur le diamètre extérieur des contacts électriques 210, ces éléments constituant des moyens de positionnement axial des contacts électriques 210 le long de la broche 20 (sur la Figure 4a, les organes de calage sont constitués par des tiges verticales 310 fixées à la platine 30 venant s'insérer dans les rainures 2112 ; la Figure 4b est une variante dans laquelle les organes de calage sont des taquets horizontaux 310' coopérant également avec les rainures 2112 périphériques des contacts électriques 210).

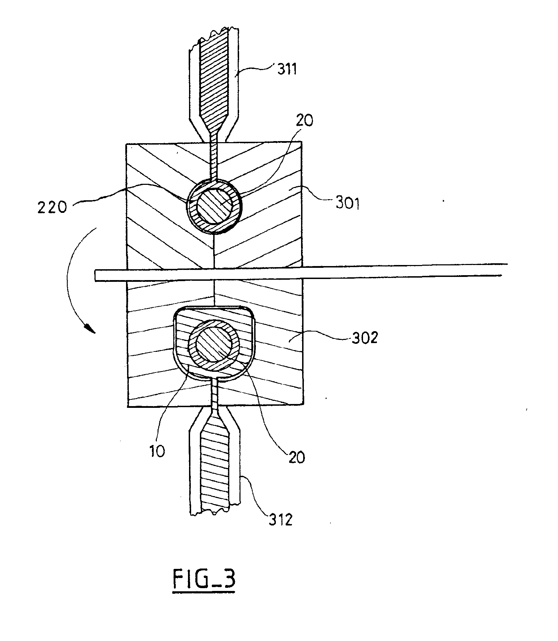

La broche 20 ainsi équipée est alors enfermée dans un premier moule 301 que l'on peut voir à la Figure 3.

Sur cette figure, les moyens de positionnement axial ne sont pas représentés. Ce premier moulage a pour but de former les joints 220 par injection, à travers la buse 311, du matériau élastique souple autour des sections profilées 22 de la broche 20. Ce matériau peut être par exemple un polymère biomédical tel qu'un *Elast-Eon* ou un *ECSil* (marques déposées) de AorTech Biomaterials, de dureté inférieure à 80 Shore A.

On obtient alors la broche 20 telle que représentée sur la Figure 4a, c'est-à-dire munie des joints 220.

Il faut souligner qu'à ce stade du procédé la coaxialité des contacts électriques 210 avec les joints 220 est garantie du fait que ces éléments sont portés par la même broche 20 de moulage. Le positionnement axial correct des joints 220 est assuré par la position définie une fois pour toutes des sections profilées sur la broche 20. De même, le positionnement axial des contacts électriques 210 est parfaitement maîtrisé grâce à l'utilisation des moyens de positionnement décrits plus haut.

Il faut également souligner le fait qu'à chaque interface joint/contact électrique, la polymérisation du matériau élastique souple crée dans un plan radial une liaison étanche entre le joint 220 et la face latérale correspondante du contact électrique 210. Ceci empêchera par la suite, lors du surmoulage de l'ensemble par un matériau rigide, toute pénétration de ce matériau entre le contact électrique 210 et le joint 220, ce qui autrement pourrait créer un chemin de fuite et de claquage électrique entre contacts électriques adjacents.

Comme l'indique la Figure 3, le moule 301 d'injection du premier matériau élastique est ensuite remplacé par le moule 302 dans lequel est injecté, à travers la buse 312, le second matériau rigide pour former la cavité 11 et la tête 10 de connecteur. Ce matériau peut être par exemple un *Elast-Eon* ou un *ECSil,* de dureté supérieure ou égale à 70 Shore D.

Les Figures 4a et 4b montrent plus en détail l'installation de la broche 20 dans le moule 302. On remarquera la présence d'une tige 320 vissée sur les composants de moulage de l'insert 100 à vis et destinée à créer une épargne pour l'introduction ultérieure de la vis 120 de serrage.

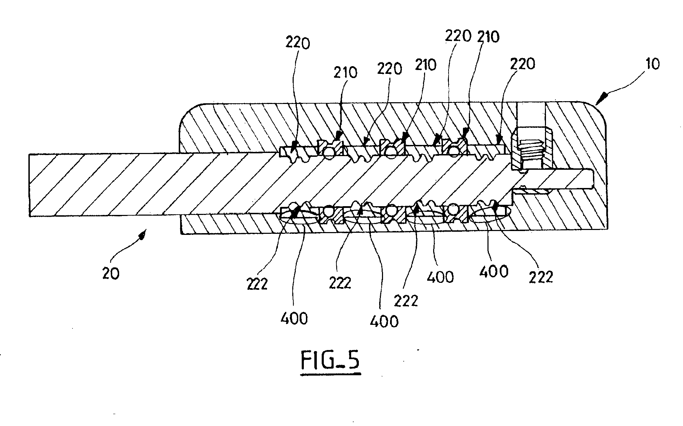

Après retrait des tiges 320 et du moule 302, on obtient la tête 10 de connecteur représentée sur la Figure 5 (la broche 20 étant encore présente). On peut voir sur cette figure que le positionnement axial des joints 220 est définitivement figé, dans la mesure où ils sont ancrés dans le matériau rigide de la tête 10.

Il en est de même pour le positionnement axial des contacts électriques 210, dont le maintien résulte du fait que chaque contact électrique se trouve enserré entre deux parois de matériau rigide. Pour obtenir ce résultat, le premier moule 301 est dimensionné de sorte que le diamètre extérieur des joints 220 soit inférieur au diamètre extérieur des contacts électriques 210.

La broche 20 peut être dégagée par déformation élastique des anneaux d'étanchéité 222.

Il faut noter que les organes 310, 310' de calage des contacts électriques 210 restent en place durant le second moulage de matériau rigide. Ils sont ensuite retirés latéralement, ce qui laisse subsister des fenêtres qui, dans le cas de Figure 4b, sont bouchées par injection de silicone. Dans le cas de la Figure 4a, les tiges 310 sont utilisées pour réaliser les prises de contact électrique avec les bagues conductrices du connecteur.

Enfin, on peut observer sur la Figure 5 la formation de zones d'adhésion chimique 400 à l'interface entre les matériaux souple et rigide injectés : ces zones d'adhésion permettent d'assurer l'étanchéité avec le milieu extérieur et l'isolation des contacts électriques entre eux et avec le milieu extérieur en faisant barrière au courant de fuite.

## Revendications

1. Un procédé de réalisation d'une tête de connecteur (10) pour un générateur de dispositif médical implantable actif, la tête de connecteur comprenant une cavité (11) sensiblement cylindrique destinée à recevoir une sonde multipolaire du dispositif, cette cavité comportant une alternance (200) d'éléments annulaires de contact électrique (210) et d'éléments annulaires d'isolement (220) présentant un profil intérieur (222) d'étanchéité,
ce procédé étant un procédé de bi-injection comprenant des étapes consistant à :
- fournir une broche cylindrique (20) de diamètre sensiblement égal au diamètre de la cavité (11), la broche comportant alternativement des sections lisses (21) destinées à recevoir les éléments annulaires de contact électrique (210), et des sections profilées (22) portant un profil conjugué du profil (222) d'étanchéité des éléments annulaires d'isolement (220) à réaliser ;
**caractérisé en ce que** le procédé comprend des étapes consistant à:
- disposer les éléments de contact électrique (210) sur les sections lisses (21) de la broche en laissant subsister axialement entre ces éléments de contact électrique un intervalle correspondant auxdites sections profilées destinées à la réalisation des éléments annulaires d'isolement ;
- injecter dans un premier moule (301) un premier matériau élastique autour des sections profilées (22) de manière à réaliser les éléments (220) d'isolement ;
- injecter dans un second moule (302) un second matériau, de dureté supérieure à celle du premier matériau, autour de la broche de manière à ancrer définitivement les éléments de contact électrique et réaliser la tête de connecteur (10) ; et
- dégager axialement la broche d'avec la cavité (11) de la tête de connecteur ainsi obtenue.

2. Le procédé de la revendication 1, comportant une étape consistant, avant injection du premier matériau, à amener les éléments (210) de contact électrique dans une position axiale déterminée sur les sections lisses (21) de la broche (20), et à les maintenir par des moyens de positionnement (2112, 310 ; 310'), cette position étant maintenue durant les étapes d'injection.

3. Le procédé de la revendication 2, dans lequel les moyens de positionnement comprennent des rainures annulaires (2112) formées sur le diamètre extérieur des éléments de contact électrique (210) et des organes (310 ; 310') de calage en position des éléments de contact électrique, aptes à coopérer avec les rainures annulaires.

4. Le procédé de la revendication 1, dans lequel le premier moule (301) d'injection est dimensionné de sorte que le diamètre extérieur des éléments d'isolement (220) soit inférieur au diamètre extérieur des éléments de contact électrique (210).

5. Le procédé de la revendication 1, dans lequel le premier matériau élastique présente une dureté inférieure à 80 Shore A.

6. Le procédé de la revendication 1, dans lequel le premier matériau élastique est un matériau élastomère ou un polymère thermoplastique.

7. Le procédé de la revendication 6, dans lequel le matériau élastomère est un composé silicone.

8. Le procédé de la revendication 6, dans lequel le polymère thermoplastique est un polyuréthane thermoplastique.

9. Le procédé de la revendication 1, dans lequel le second matériau a une dureté supérieure ou égale à 70 Shore D.

10. Le procédé de la revendication 1, dans lequel le second matériau est un polymère thermoplastique.

11. Le procédé de la revendication 10, dans lequel le polymère thermoplastique est un polyuréthane thermoplastique.

## Patentansprüche

1. Verfahren zum Herstellen eines Verbinderkopfes (10) für einen Generator einer aktiven implantierbaren medizinischen Vorrichtung, wobei der Verbinderkopf einen im Wesentlichen zylindrischen Hohlraum (11) umfasst, der dazu bestimmt ist, eine multipolare Sonde der Vorrichtung aufzunehmen, wobei dieser Hohlraum eine abwechselnde Folge (200) von ringförmigen Elementen (210) für elektrischen Kontakt und von ringförmigen Isolationselementen (220), die ein Dichtungsinnenprofil (222) aufweisen, umfasst, wobei das Verfahren ein Biinjektionsverfahren ist, das die Schritte umfasst, die darin bestehen:
- einen zylindrischen Stift (20) mit einem Durchmesser vorzusehen, der im Wesentlichen gleich dem Durchmesser des Hohlraums (11) ist, wobei der Stift abwechselnd glatte Abschnitte (21), die dazu bestimmt sind, die ringförmigen Elemente (210) für elektrischen Kontakt aufzunehmen, und Profilabschnitte (22), die ein zu dem herzustellenden Dichtungsprofil (222) der ringförmigen Isolationselemente (220) konjugiertes Profil tragen, umfasst;
**dadurch gekennzeichnet, dass** das Verfahren die folgenden Schritte umfasst, die darin bestehen:
- die Elemente (210) für elektrischen Kontakt auf den glatten Abschnitten (21) des Stifts anzuordnen und dabei axial zwischen diesen Elementen für elektrischen Kontakt ein Intervall freizulassen, das den Profilabschnitten entspricht, die für die Herstellung der ringförmigen Isolationselemente bestimmt sind;
- in eine erste Form (301) ein erstes elastisches Material um die Profilelemente (22) einzuspritzen, derart, dass die Isolationselemente (220) verwirklicht werden;
- in eine zweite Form (302) ein zweites Material mit höherer Härte als jene des ersten Materials um den Stift in der Weise einzuspritzen, dass die Elemente für elektrischen Kontakt endgültig verankert werden und der Verbinderkopf (10) verwirklicht wird; und
- den Stift aus dem Hohlraum (11) des somit erhaltenen Verbinderkopfes axial herauszuziehen.

2. Verfahren nach Anspruch 1, das einen Schritt umfasst, der darin besteht, vor dem Einspritzen des ersten Materials die Elemente (210) für elektrischen Kontakt in eine bestimmte axiale Position auf den Plattenabschnitten (21) des Stifts (20) zu bringen und sie durch Positionierungsmittel (2112, 310; 310') zu halten, wobei diese Position während der Einleitungsschritte beibehalten wird.

3. Verfahren nach Anspruch 2, wobei die Positionierungsmittel ringförmige Nuten (2112), die auf dem Außendurchmesser der Elemente (210) für elektrischen Kontakt gebildet sind, und Organe (310; 310') zum Verkeilen der Elemente für elektrischen Kontakt an ihrer Position, die mit den ringförmigen Nuten zusammenwirken können, umfassen.

4. Verfahren nach Anspruch 1, wobei die erste Einspritzform (301) so bemessen ist, dass der Außendurchmesser der Isolationselemente (220) kleiner ist als der Außendurchmesser der Elemente (210) für elektrischen Kontakt.

5. Verfahren nach Anspruch 1, wobei das erste elastische Material eine Härte von weniger als 80 Shore A aufweist.

6. Verfahren nach Anspruch 1, wobei das erste elastische Material ein Elastomermaterial oder ein thermoplastisches Polymer ist.

7. Verfahren nach Anspruch 6, wobei das Elastomermaterial eine Silikonverbindung ist.

8. Verfahren nach Anspruch 6, wobei das thermoplastische Polymer ein thermoplastisches Polyurethan ist.

9. Verfahren nach Anspruch 1, wobei das zweite Material eine Härte größer oder gleich 70 Shore D hat.

10. Verfahren nach Anspruch 1, wobei das zweite Material ein thermoplastisches Polymer ist.

11. Verfahren nach Anspruch 10, wobei das thermoplastische Polymer ein thermoplastisches Polyurethan ist.

## Claims

1. A method of making a connector head (10) for a generator of an active implantable medical device, the connector head comprising a substantially cylindrical cavity (11) intended to receive a multipolar lead of the device, this cavity including an alternation (200) of electric-contact annular elements (210) and of isolation annular elements (220) having an inner tightness profile (222), this method being a bi-injection method comprising the steps of:
- providing a cylindrical pin (20) of diameter substantially equal to the diameter of the cavity (11), the pin including alternately smooth sections (21) intended to receive the electric-contact annular elements (210), and profiled sections (22) having a profile mating with the tightness profile (222) of the isolation annular elements (220) to be made;
**characterized in that** the method comprises the steps of:
- placing the electric contact elements (210) on the smooth sections (21) of the pin, while leaving between these electric contact elements an axial interval corresponding to said profiled sections intended to the making of the isolation annular elements;
- injecting in a first mold (301) a first elastic material around the profiled sections (22) so as to make the isolation elements (220) ;
- injecting in a second mold (302) a second material, of hardness higher than that of the first material, around the pin, so as to definitively anchor the electric contact elements and to make the connector head (10) ; and
- axially release the pin from the cavity (11) of the thus-obtained connector head.

2. The method of claim 1, including a step of, before injecting the first material, bringing the electric contact elements (210) in a determined axial position on the smooth sections (21) of the pin (20), and maintaining them by positioning means (2112, 310 ; 310'), this position being maintained during the steps of injection.

3. The method of claim 2, wherein the positioning means comprise annular grooves (2112) formed on the external diameter of the electric contact elements (210) and members (310; 310') for fastening in position the electric contact elements, adapted to cooperate with the annular grooves.

4. The method of claim 1, wherein the first injection mold (301) is sized in such a manner that the external diameter of the isolation elements (220) is smaller than the external diameter of the electric contact elements (210).

5. The method of claim 1, wherein the first elastic material has a hardness lower than 80 Shore A.

6. The method of claim 1, wherein the first elastic material is an elastomeric material or a thermoplastic polymer.

7. The method of claim 6, wherein the elastomeric material is a silicon compound.

8. The method of claim 6, wherein the thermoplastic polymer is a thermoplastic polyurethane.

9. The method of claim 6, wherein the second material has a hardness higher than or equal to 70 Shore D.

10. The method of claim 1, wherein the second material is a thermoplastic polymer.

11. The method of claim 10, wherein the thermoplastic polymer is a thermoplastic polyurethane.
